(19) Europäisches Patentamt / European Patent Office / Office européen des brevets

(11) **EP 3 815 705 A1**

(12) **EUROPEAN PATENT APPLICATION**
published in accordance with Art. 153(4) EPC

(43) Date of publication:
**05.05.2021 Bulletin 2021/18**

(51) Int Cl.:
*A61K 38/08* (2019.01)    *A61K 31/7068* (2006.01)
*A61P 35/00* (2006.01)    *A61P 35/04* (2006.01)

(21) Application number: **19819254.4**

(22) Date of filing: **13.06.2019**

(86) International application number:
**PCT/KR2019/007117**

(87) International publication number:
**WO 2019/240505 (19.12.2019 Gazette 2019/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.06.2018 US 201862685019 P**

(71) Applicant: **At-Pharma Co.,Ltd.**
**Seongnam-si, Gyeonggi-do 13486 (KR)**

(72) Inventors:
• **KANG, Jae Seung**
  **Seongnam-si, Gyeonggi-do 13486 (KR)**
• **LEE, Wang Jae**
  **Seongnam-si, Gyeonggi-do 13486 (KR)**

(74) Representative: **Klunker IP**
**Patentanwälte PartG mbB**
**Destouchesstraße 68**
**80796 München (DE)**

(54) **COMPOSITION AND THERAPEUTIC ADJUVANT FOR TREATING PANCREATIC CANCER, BOTH CONTAINING ALLOFERON**

(57) A compound of structural formula (1) exhibits, in the treatment of cancer, unexpected advantages of excellent efficacy and reduced side effects. In particular, excellent efficacy in adjuvant therapy for some specific cancers is exhibited. Specifically, a method for treating pancreatic cancer by using a compound of structural formula (1) alone or in combination is disclosed. [Structural formula 1] $X_1$-His-Gly-$X_2$-His-Gly-Val-$X_3$ (In structural formula (1), $X_1$ is not present or represents at least one amino acid residue, $X_2$ represents a peptide bond or at least one amino acid residue and $X_3$ is not present or represents at least one amino acid residue or a physiologically acceptable salt.)

[FIG.8]

EP 3 815 705 A1

```
<110>    AT-Pharma Co., Ltd.

<120>    COMPOSITION FOR ANTI-PANCREATIC CANCER AND ANTI-PANCREATIC
         ADJUVANT COMPRISING ALLOFERON

<130>    20190610

<150>    US 62/685,019
<151>    2018-06-14

<160>    20

<170>    KoPatentIn 3.0

<210>    1
<211>    13
<212>    PRT
<213>    Calliphora vicina

<400>    1
His Gly Val Ser Gly His Gly Gln His Gly Val His Gly
 1               5                   10


<210>    2
<211>    12
<212>    PRT
<213>    Calliphora vicina

<400>    2
Gly Val Ser Gly His Gly Gln His Gly Val His Gly
 1               5                   10


<210>    3
<211>    10
<212>    PRT
<213>    Calliphora vicina

<400>    3
Val Ser Gly His Gly Gln His Gly Val His
 1               5                   10


<210>    4
<211>    8
<212>    PRT
<213>    Calliphora vicina

<400>    4
Ser Gly His Gly Gln His Gly Val
 1               5


<210>    5
<211>    13
<212>    PRT
<213>    Calliphora vicina

<400>    5
Pro Ser Leu Thr Gly His Gly Phe His Gly Val Tyr Asp
 1               5                   10
```

```
<210>    6
<211>    11
<212>    PRT
<213>    Calliphora vicina

<400>    6
Phe Ile Val Ser Ala His Gly Asp His Gly Val
1               5                   10


<210>    7
<211>    7
<212>    PRT
<213>    Calliphora vicina

<400>    7
Thr His Gly Gln His Gly Val
1               5


<210>    8
<211>    7
<212>    PRT
<213>    Calliphora vicina

<400>    8
His Gly His Gly Val His Gly
1               5


<210>    9
<211>    10
<212>    PRT
<213>    Calliphora vicina

<400>    9
Leu Ala Ser Leu His Gly Gln His Gly Val
1               5                   10


<210>    10
<211>    13
<212>    PRT
<213>    Calliphora vicina

<400>    10
Cys Val Val Thr Gly His Gly Ser His Gly Val Phe Val
1               5                   10


<210>    11
<211>    10
<212>    PRT
<213>    Calliphora vicina

<400>    11
Ile Ser Gly His Gly Gln His Gly Val Pro
1               5                   10


<210>    12
<211>    9
```

```
<212>     PRT
<213>     Calliphora vicina

<400>     12
Cys Gly His Gly Asn His Gly Val His
  1               5


<210>     13
<211>     12
<212>     PRT
<213>     Calliphora vicina

<400>     13
Ile Val Ala Arg Ile His Gly Gln Asn His Gly Val
  1               5                       10


<210>     14
<211>     11
<212>     PRT
<213>     Calliphora vicina

<400>     14
His Gly Ser Asp Gly His Gly Val Gln His Gly
  1               5                   10


<210>     15
<211>     7
<212>     PRT
<213>     Calliphora vicina

<400>     15
Phe Gly His Gly His Gly Val
  1               5


<210>     16
<211>     8
<212>     PRT
<213>     Calliphora vicina

<400>     16
His Gly Asn His Gly Val Leu Ala
  1               5


<210>     17
<211>     12
<212>     PRT
<213>     Calliphora vicina

<400>     17
His Gly Asp Ser Gly His Gly Gln His Gly Val Asp
  1               5                       10


<210>     18
<211>     7
<212>     PRT
<213>     Calliphora vicina
```

```
<400>      18
His Gly His Gly Val Pro Leu
 1                   5


<210>      19
<211>      10
<212>      PRT
<213>      Calliphora vicina

<400>      19
Ser Gly His Gly Ala Val His Gly Val Met
 1                   5                   10


<210>      20
<211>      12
<212>      PRT
<213>      Calliphora vicina

<400>      20
Tyr Ala Met Ser Gly His Gly His Gly Val Phe Ile
 1                   5                   10
```

**Description**

[Technical Field]

The present disclosure generally relates to the use of peptides (alloferons) of formula (1).

[formula 1]     $X_1$-His-Gly-$X_2$-His-Gly-Val-$X_3$

(In formula (1), wherein the groups $X_1$ to $X_3$ have the meanings given in the claims in cancer therapy.)

[Background Art]

The compounds of formula (1) have been shown to immunomodulate anticancer activity in animal. In particular, an alloferon has been shown to stimulate the natural cytotoxicity of human peripheral blood lymphocytes, induce interferon synthesis in mouse and human models, and enhance antiviral and antitumor resistance in mice. (Chernysh, Proceedings of the National Academy of Science of the United States 99, 12628 (2002)). It has been suggested that alloferons have a therapeutic effect similar to interferons, but the chemical structure of alloferon do not share any similarity with interferons, other known cytokines and interferon inducers as well as any other materials of medical importance.. (U.S. Pat. Nos. 6,692,747).

Alloferons have been shown to enhance natural killer (NK) cell cytotoxicity. (Bae, Immunobiology 218(8), 1026 (2013)). NK cells are re a type of lymphocyte and a component of innate immune system. NK cells are known to play a key role in antitumor and antiviral immunity. Bae suggests that alloferon has antitumor effects through up-regulation of NK-activating receptor 2B4 and the enhancement of granule exocytosis from NK cells.

Peptides of formula (1), $X_1$-His-Gly-$X_2$-His-Gly-Val-$X_3$, wherein $X_1$ is absent or represents at least one amino acid residue, $X_2$ is a peptide bond or represents at least one amino acid residue, and $X_3$ is absent or represents at least one amino acid residue are disclosed in U.S. Pat. Nos. 6,692,747 and 7,462,360 as biologically active peptides specifically stimulating antiviral, antimicrobial, and antitumor activity of the human and animal immune system. These patents also disclose methods of manufacturing a composition having immunomodulatory activity, comprising combining a peptide of formula (1). The disclosure of U.S. Pat. Nos. 6,692,747 and 7,462,360 and WO 2005/037,824 are incorporated by reference.

Each cancer therapeutic carries with it a risk of a serious side effect. For example, paclitaxel has been associated with a number of clinical toxicities. (Rowinsky, Seminars in Oncology 20 (4), 1 (1993)). In addition, gemcitabine, a therapeutic used in the treatment of pancreatic AC, one of the most aggressive solid cancers, is associated with hematological toxicities. (Xie, Diagnostic Pathology 9, 214 (2014)). Thus, there is a need for antitumor treatments that are efficacious in treating cancers such as those identified above while reducing or minimizing the risk of side effects of an antitumor therapeutic agent.

[Related Art References]

[Patent literatures]

(Patent Literature 1) US6692747 B2
(Patent Literature 2) US7462360 B2
(Patent Literature 3) WO2005037824 A2

[Non-Patent literatures]

(Non-Patent Literature 1) Chernysh, Proceedings of the National Academy of Science of the United States 99, 12628 (2002)
(Non-Patent Literature 2) Bae, Immunobiology 218(8), 1026 (2013)
(Non-Patent Literature 3) Rowinsky, Seminars in Oncology 20 (4), 1 (1993)
(Non-Patent Literature 4) Xie, Diagnostic Pathology 9, 214 (2014)

[Disclosure]

[Technical Problem]

A first aspect of the present invention therefore is a method of treating cancer, preferably the specific cancer-subindications referred to hereinafter, said method comprising administering a therapeutically effective amount of a compound of formula (1) to a patient in need thereof, optionally in combination with inhibitors of metabolic processes (e.g., gemcitabine, 5-FU), topoisomerase inhibitors (e.g., etoposide), cytokines (e.g., interferons), mitosis inhibitors (e.g., vinblastine, paclitaxel), compounds which interact with nucleic acids (e.g., cis-platin, cyclophosphamide, adriamycin), hormone antagonists (e.g., tamoxifen), or antibodies (e.g., nivolumab, ipilimumab) and/or tumor resection by surgery.
A second aspect of the present invention is directed to the use of a compound of formula (1) for the manufacture of a medicament for the treatment of cancer, preferably for the treatment of the specific cancer-subindications referred to hereinafter.

[Technical Solution]

According to one aspect of the present invention, a pharmaceutical composition for reducing metastasis and treating pancreatic cancer comprising a compound of formula (1) is provieded.

[formula 1]    $X_1$-His-Gly-$X_2$-His-Gly-Val-$X_3$

(In formula (1),
wherein $X_1$ is absent or represents at least one amino acid residue, $X_2$ is a peptide bond or represents at least one amino acid residue, and $X_3$ is absent or represents at least one amino acid residue, or a physiologically acceptable salt thereof.)
Also, the compound of formula (1) may comprise peptides having at least one sequence selected from the group consisting of SEQ ID NO:1 to SEQ ID NO:20.
Also, the composition can be administered in combination with one or more inhibitor of metabolic processes, topoisomerase inhibitor, cytokine, mitosis inhibitor, compound which interacts with nucleic acids, hormone antagonist, or antibody, a combination of any of said antitumor therapeutic agents.
Also, the composition may improve the anti-cancer activity of gemcitabine.
According to other aspect of the present invention, an adjuvant for treating pancreatic cancer comprising a compound of formula (1) is provided.

[formula 1]    $X_1$-His-Gly-$X_2$-His-Gly-Val-$X_3$

(In formula (1),
wherein $X_1$ is absent or represents at least one amino acid residue, $X_2$ is a peptide bond or represents at least one amino acid residue, and $X_3$ is absent or represents at least one amino acid residue, or a physiologically acceptable salt thereof.)
Also, the adjuvant can be intended to be combined with tumor therapy.
Also, the tumor therapy may be one or more treating antitumor therapeutic agents or tumor resection by surgery, a combination thereof.
Also, the antitumor therapeutic agent may be one or more inhibitor of metabolic processes, topoisomerase inhibitor, cytokine, mitosis inhibitor, compound which interacts with nucleic acids, hormone antagonist, or antibody, a combination of any of said antitumor therapeutic agents.
Also, the antitumor therapeutic agent may be gemcitabine.
Also, the compound of formula (1) may comprise peptides having a sequence selected from the group consisting of SEQ ID NO: 1 to SEQ ID NO:20.

[Advantageous Effects]

It has been found that the compounds of formula (1) provide unexpected advantages in the treatment of cancer, e.g., superior efficacy and/or reduced side effects. The superior efficacy and reduced side effects are seen especially in the treatment of several specific cancer-subindications.

[Description of Drawings]

FIG. 1 illustrates molecular pathways involved in cancer cell metastasis.

FIG. 2(a) illustrates a reduction of expression of CXCR4 in lung cancer cells treated with alloferon, FIG. 2(b) illustrates a reduction of stromal cell derived factor 1 (SDF-1) production in A549 cells treated with alloferon, and FIG 2(c) illustrates a reduction of stromal cell derived factor 1 (SDF-1) production in H1299 cells treated with alloferon.

FIG. 3(a) shows a suppression of migration in A549 cells treated with alloferon, and FIG. 3(b) shows a suppression of migration in H1299 cells treated with alloferon.

FIG. 4 shows that a combined treatment with alloferon and gemcitabine increases apoptosis of pancreatic cancer cells.

FIG. 5 shows the cell cycle distribution of pancreatic cancer cells concomitantly treated with alloferon and gemcitabine.

FIG. 6 shows a mitochondrial membrane potential assay for pancreatic cancer cells concomitantly treated with alloferon and gemcitabine.

FIG. 7 shows the suppression of pancreatic cancer cell growth concomitantly treated with alloferon and gemcitabine.

FIG. 8 shows tumor burdens from each experimental groups and suppressive effect of alloferon and gemcitabine on tumor growth.

[Mode for Invention]

In the following description, numerous specific details are set forth in order to provide a thorough understanding of the embodiments of the present disclosure. However, it will be apparent to those skilled in the art that the embodiments, including structures, systems, and methods, may be practiced without these specific details. The description and representation herein are the common means used by those experienced or skilled in the art to most effectively convey the substance of their work to others skilled in the art. In other instances, well-known methods, procedures, components, and circuitry have not been described in detail to avoid unnecessarily obscuring embodiments of the disclosure.

The accompanying drawings, which are incorporated herein and form a part of the specification, illustrate the embodiments of the present disclosure and, together with the description, further serve to explain the principles of the embodiments and to enable a person skilled in the pertinent art to make and use the embodiments.

The indication "cancer" as used in the context of the invention is to be understood in a most general sense as a disease characterized by inappropriate cellular proliferation, migration, apoptosis and/or angiogenesis. Inappropriate cell proliferation means cellular proliferation resulting from inappropriate cell growth, from excessive cell division, from cell division at an accelerated rate and/or from inappropriate cell survival.

In a first embodiment (1), both with regard to the first and second aspect of the invention, formula (1) is expressed as below.

[formula 1]     $X_1$-His-Gly-$X_2$-His-Gly-Val-$X_3$

Formula (1) is defined to encompass those compounds wherein
$X_1$ is absent or represents at least one amino acid residue, $X_2$ is a peptide bound or represents at least one amino acid residue, and $X_3$ is absent or represents at least one amino acid residue, or a pharmaceutically acceptable salt or ether thereof, the peptide exhibiting immunomodulatory activity.

Compounds of formula (1) include, but are not limited to, peptides having a sequence selected from the group consisting of SEQ ID NO:1 to SEQ ID NO:20. In a preferred embodiment, compounds of formula (1) may include peptides having the the amino acid sequence of SEQ ID NO:1 or SEQ ID NO:2. In an especially preferred embodiment,-compounds of formula (1) may include peptides having the amino acid sequence of SEQ ID NO:2. In describing one embodiment, the compounds of the above formula (1) is referred to as the 'alloferon'. In detailed, alloferons may, but are not limited to, peptides having the sequence of SEQ ID NO:1 or SEQ ID NO:2. In more detailed, alloferons may peptides having the sequence of SEQ ID NO:2. Alloferons may be obtained by separating and refining from nature or by synthesizing it.

In addition to the peptides described herein, peptidomimetics are also contemplated. Peptide analogs are commonly used in the pharmaceutical industry as non-peptide drugs with properties analogous to those of the template peptide. Such peptidomimetics include chemically modified peptides, peptide- like molecules containing non-naturally occurring amino acids, and peptoids. These types of non-peptide compound are termed "peptide mimetics" or "peptidomimetics" and are usually developed with the aid of computerized molecular modeling. Peptide mimetics that are structurally similar to therapeutically useful peptides may be used to produce an equivalent therapeutic or prophylactic effect.

The peptide or peptidomimetic may be cyclic or otherwise conformationally constrained. Conformationally constrained molecules can have improved properties such as increased affinity, metabolic stability, membrane permeability or solubility. Methods of conformational constraint are well known in the art.

Compositions comprising compounds of formula (1) may be prepared. The composition according to the invention for parenteral administration is generally in the form of a solution or suspension of the peptide in a pharmaceutically acceptable carrier, preferably an aqueous carrier. Examples of aqueous carriers that may be used include water, buffered water, saline solution (0.4%), glycine solution (0.3%), hyaluronic acid and similar known carriers. Apart from aqueous carriers it is also possible to use solvents such as dimethylsulphoxide, propyleneglycol, dimethylformamide and mixtures thereof. The composition may also contain pharmaceutically acceptable excipients such as buffer substances and inorganic salts in order to achieve normal osmotic pressure and/or effective lyophilization. Examples of such additives are sodium and potassium salts, e.g., chlorides and phosphates, sucrose, glucose, protein hydrolysates, dextran, polyvinylpyrrolidone or polyethylene glycol. The compositions may be sterilized by conventional methods, e.g., by sterile filtration. The composition may be decanted directly in this form or lyophilized and mixed with a sterile solution before use.

Depending on the type and severity of the disease, about 1 $\mu$g/kg to 1000 mg/kg of body weight once per day of the compound of formula (1) is an initial candidate dosage for administration to the patient, whether, for example, by one or more separate administrations, or by continuous infusion. A typical daily dosage might range from about 1 $\mu$g/kg to 100 mg/kg or more, depending on the factors mentioned above, more preferably about 0.1 to 20 mg/kg of body weight, and, when administered subcutaneously or intramuscularly, about 1 to 20 mg/kg of body weight. Necessary modifications in this dosage range may be determined by one of ordinary skill in the art using only routine experimentation given the teachings herein. (Remington's Pharmaceutical Sciences, 20th edition, (ed. A. Gennaro; Lippincott, Williams & Wilkins 2000)). For repeated administrations over several days or longer, depending on the condition, the treatment is sustained until a desired suppression of disease symptoms occurs. However, other dosage regimens may be useful. The progress of the therapy according to the invention is easily monitored by conventional techniques and assays.

Compositions comprising compounds of formula (1) may be administered in a number of ways. Representative delivery regimens include oral, subcutaneous, parenteral (including subcutaneous, intramuscular and intravenous injection), rectal, buccal (including sublingual), transdermal, inhalation ocular and intranasal.

In one embodiment, delivery of peptides entails subcutaneous injection of a controlled-release injectable formulation. In some embodiments, peptides and/or proteins described herein are useful for subcutaneous, intranasal and inhalation administration.

In a preferred embodiment the invention relates to the use of a compound of formula (1) according to the invention, wherein the disease is cancer selected from the group consisting of carcinomas, sarcomas, melanomas, myelomas, hematological neoplasias, lymphomas and childhood cancers.

Examples of carcinomas within the scope of the invention include but are not limited to adenocarcinoma (AC), squamous cell carcinoma (SCC) and mixed or undifferentiated carcinomas. Carcinomas within the scope of the invention include but are not limited to the following histologies:

Head and neck tumors: SCC, AC, transitional cell cancers, mucoepidermoid cancers, undifferentiated carcinomas;
Central nervous system tumors: Astrocytoma, glioblastoma, meningeoma, neurinoma, schwannoma, ependymoma, hypophysoma, oligodendroglioma, medulloblastoma;
Bronchial and mediastinal tumors:
Bronchial tumors:

Small cell lung cancers (SCLC): oat-cell lung cancer, intermediate cell cancer, combined oat-cell lung cancer;
Non-small cell lung cancers (NSCLC): SCC, spindle cell carcinoma, AC, bronchioalveolar carcinoma, large cell NSCLC, clear cell NSCLC;
Mesothelioma;
Thymoma;
Thyroid carcinomas: papillary, follicular, anaplastic, medullary;
Tumors of the gastrointestinal tract:
Esophageal cancers: SCC, AC, anaplastic, carcinoid, sarcoma;
Gastric cancers: AC, adenosquamous, anaplastic;
Colorectal cancers: AC, including hereditary forms of AC, carcinoid, sarcoma;
Anal cancers: SCC, transitional epithelial cancer, AC, basal cell carcinoma;
Pancreatic cancers: AC, including ductal and acinary cancers, papillary, adenosquamous, undifferentiated, tumors of the endocrine pancreas;
Hepatocellular carcinoma, cholangiocarcinoma, angiosarcoma, hepatoblastoma;
Biliary carcinomas: AC, SCC, small cell, undifferentiated;
Gastrointestinal stroma tumors (GIST);

Gynecological cancers:

Breast cancers: AC, including invasive ductal, lobular and medullary cancers, tubular, mucinous cancers, Paget-carcinoma, inflammatory carcinoma, ductal and lobular carcinoma in situ;

Ovarian cancers: Epithelial tumors, stroma tumors, germ cell tumors, undifferentiated tumors;

Cervical cancers: SCC, AC, mixed and undifferentiated tumors;

Endometrial cancers: AC, SCC, mixed, undifferentiated tumors;

Vulvar cancers: SCC, AC;

Vaginal cancers: SCC, AC;

Urinary tract and testicular cancers:

Testicular cancers: seminoma;

Non-seminomatous germ cell tumors: teratoma, embryonal cell carcinoma, choriocarcinoma, yolk sac tumor, mixed, Sertoli and Leydig-cell tumors;

Extragonadal germ cell tumors;

Prostate cancers: AC, small cell, SCC;

Renal cell cancers: AC, including clear cell, papillary and chromophobous carcinomas, hereditary forms (e.g., von-Hippel-Lindau syndrome), nephroblastoma;

Urinary bladder cancers: transitional cell (urothelial) cancers, SCC, AC;

Urethral cancers: SCC, transitional cell cancers, AC;

Penile cancers: SCC;

Tumors of endocrine tissue:

Thyroid cancers: papillary, follicular, anaplastic, medullary carcinomas, including MEN (multiple endocrine neoplasia) syndromes;

Tumors of the endocrine pancreas;

Carcinoids;

Pheochromocytoma.

Examples of sarcomas within the scope of the invention include but are not limited to Ewing-sarcoma, osteosarcoma or osteogenic sarcoma, chondrosarcoma, synovial sarcoma, leiomyosarcoma, rhabdomyosarcoma, mesothelial sarcoma or mesothelioma, fibrosarcoma, angiosarcoma or hemangioendothelioma, liposarcoma, glioma or astrocytoma, myxosarcoma, malignant fibrous histiocytoma, mesenchymous or mixed mesodermal tumor, neuroblastoma and clear cell sarcoma.

Examples of melanomas within the scope of the invention include but are not limited to superficial spreading melanoma, nodular and lentigo-maligna melanoma.

Examples of myelomas within the scope of the invention include but are not limited to immunocytoma, plasmocytoma and multiple myeloma.

In another preferred embodiment the invention relates to the use according to the invention, wherein the hematological neoplasia is leukemia.

Further examples of hematologic neoplasias within the scope of the invention include but are not limited to acute or chronic leukemias of myeloid, erythroid or lymphatic origin, myelodysplastic syndromes (MDS) and myeloproliferative syndromes (MPS, such as chronic myelogeneous leukemia, osteomyelofibrosis, polycythemia vera or essential thrombocythemia).

Examples of lymphomas within the scope of the invention include but are not limited to:

Hodgkin's-lymphoma;

Non-Hodgkin's-lymphomas: T- and B-cell lymphomas

B-cell lymphomas:

Low and intermediate grade: Chronic lymphocytic leukemia (CLL), prolymphocytic leukemia (PLL), small lymphocytic lymphoma, hairy cell leukemia, plasmacytoid lymphoma, mantle cell lymphoma, follicular lymphoma, marginal zone lymphoma including MALT-lymphoma;

High grade: diffuse large B-cell lymphoma (DLBCL including immunoblastic and centroblastic variants), lymphoblastic, Burkitt's lymphoma;

T-cell lymphomas:

Low grade: T-CLL, T-PLL, Mycosis fungoides, Sezary-syndrome;

High grade: Anaplastic large cell, T-immunoblastic and lymphoblastic.

In another preferred embodiment the invention relates to the use according to the invention, wherein the disease is cancer selected from the group consisting of mixed tumors, undifferentiated tumors and metastases thereof.

Examples of mixed tumors within the scope of the invention include but are not limited to adenosquamous carcinomas, mixed mesodermal tumors, carcinosarcomas and teratocarcinomas.

Examples of undifferentiated, other tumors or metastases thereof within the scope of the invention include but are not limited to undifferentiated tumors, carcinomas of unknown primary (CUP), metastases of unknown primary (MUP) and pheochromocytoma, carcinoids.

Additionally the following tumor diseases which can be treated with a compound of formula (1) in accordance with the invention are summarized: acral lentiginous melanoma, actinic keratoses, adenoid cycstic carcinoma, adenomas, adenosarcoma, adrenocortical carcinoma, AIDS-related lymphoma, bartholin gland carcinoma, brain stem glioma, capillary carcinoma, central nervous system lymphoma, chondosarcoma, choriod plexus papilloma/carcinoma, cystadenoma, endodermal sinus tumor, endometrial hyperplasia, endometrial stromal sarcoma, endometrioid adenocarcinoma, epitheloid, focal nodular hyperplasia, gastrinoma, gestational trophoblastic tumor, glucagonoma, hepatic adenoma, hepatic adenomatosis, hypopharyngeal cancer, hypothalamic and visual pathway glioma, insulinoma, intraepithelial neoplasia, interepithelial squamous cell neoplasia, intraocular invasive squamous cell carcinoma, large cell carcinoma, islet cell carcinoma, Kaposi's sarcoma, laryngeal cancer, leukemia-related disorders, lip and oral cavity cancer, malignant mesothelial tumors, malignant thymoma, medulloepithelioma, merkel cell carcinoma, mucoepidermoid carcinoma, multiple myeloma/plasma cell neoplasm, mycosis fungoides, myelodysplastic syndrome, myeloproliferative disorders, nasal cavity and paranasal sinus cancer, nasopharyngeal cancer, neuroepithelial adenocarcinoma, nodular melanoma, oat cell carcinoma, oligodendroglial, oral cancer, oropharyngeal cancer, pineal cell, pituitary tumors, pseudosarcoma, pulmonary blastoma, parathyroid cancer, pineal and supratentorial primitive neuroectodermal tumors, pituitary tumor, plasma cell neoplasm, pleuropulmonary blastoma, retinoblastoma, serous carcinoma, small intestine cancer, soft tissue carcinomas, somatostatin-secreting tumor, supratentorial primitive neuroectodermal tumors, uveal melanoma, verrucous carcinoma, vipoma, Waldenstrom's macroglobulinemia, well differentiated carcinoma, and Wilm's tumor.

It is known for treatment of tumor diseases that the compounds may be used in monotherapy or in conjunction with other antitumor therapeutic agents or procedures, for example in combination with inhibitors of metabolic processes (e.g., gemcitabine, 5-FU), topoisomerase inhibitors (e.g., etoposide), cytokines (e.g., interferons), mitosis inhibitors (e.g., vinblastine, paclitaxel), compounds which interact with nucleic acids (e.g., cis-platin, cyclophosphamide, adriamycin), hormone antagonists (e.g., tamoxifen), or antibodies (e.g., nivolumab, ipilimumab) and/or tumor resection by surgery.

In a preferred embodiment the invention relates to the use of a compound of formula (1) to treat tumors of the pancreas. The compound of formula (1) may be used in may be used in monotherapy or in conjunction with other antitumor therapeutic agents or procedure for the treatment of tumors of the pancreas.

In another preferred embodiment the invention relates to the use of a compound of formula (1) to treat tumors of the pancreas. The compound of formula (1) may be used in may be used in monotherapy or in conjunction with other antitumor therapeutic agents or procedure for the treatment of tumors of the pancreas. In particular, a preferred embodiment of the invention relates to the use of a compound of formula (1) in combination with gemcitabine to treat tumors of the pancreas.

In order to assist in understanding the invention, the invention is described in more detail in accordance with the example below. However, these examples only illustrate this invention and do not limit the scope of the claims attached, and it is clear that various changes and modifications can be made within the categories of this invention and within the scope of technical thought, and these changes and modifications are within the scope of the claims attached.

## EXAMPLES

<EXAMPLE 1>

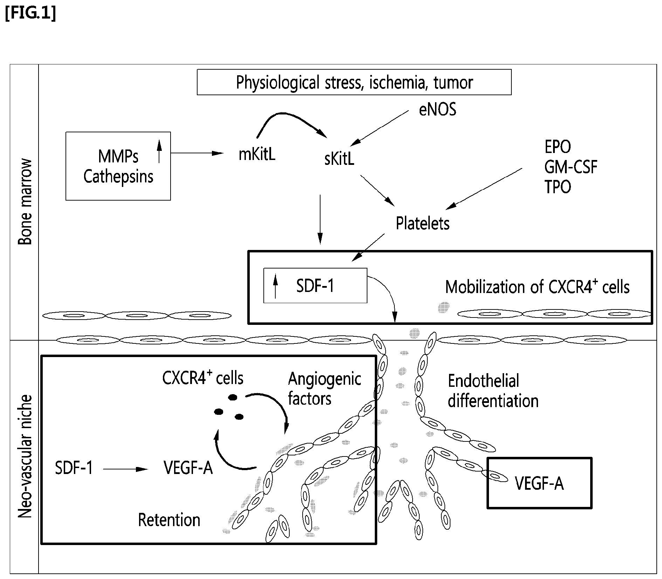

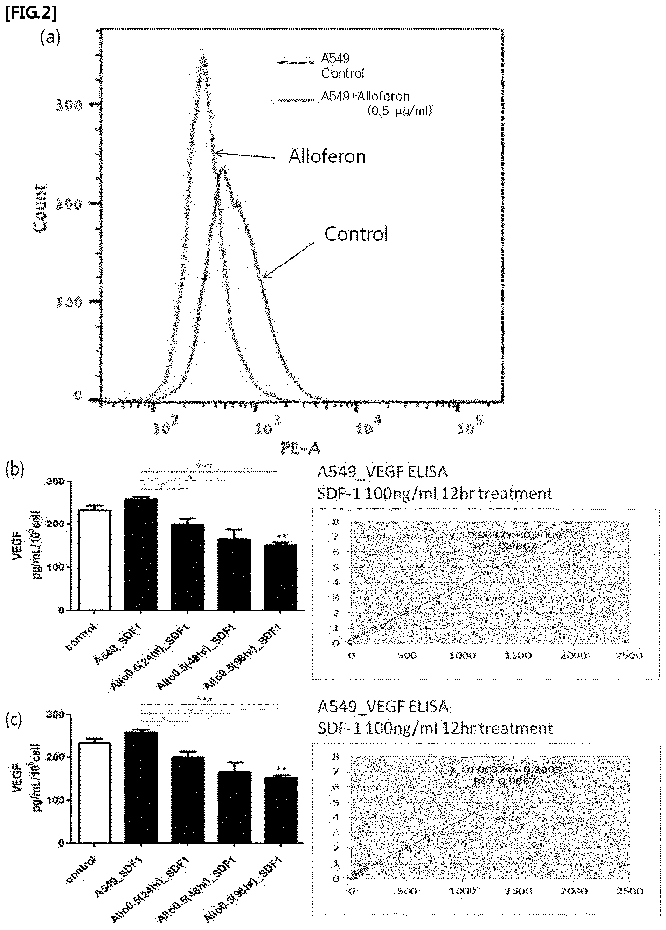

As shown in FIG. 1, chemokine receptor type 4 or fusin (CXCR4), which is expressed on the surface of cancer cells, binds to stromal-derived factor 1 (SDF-1) and induces expression and production of factors involved in cancer cell metastasis, such as VEGF. When alloferon was administered to non-small cell lung cancer cell line A549 (FIG. 2(a)), the expression of CXCR4 on the cell surface of the A549 cells decreased as compared to control A549 cells. One hundred thousand A549 cells, a non-small cell lung cancer cell line, were pre-treated 0.5 $\mu$g/ml of alloferon for 96 hours, and then cells were collected and washed three times with cold phosphate-buffered saline (PBS). Cells were resuspended with 300 $\mu$l of cold PBS and 5 $\mu$l of PE-conjugated anti-human CXCR4 monoclonal antibody (BD Biosciences) was added. After 30 minute on ice, the cells were washed, and then CXCR4 expression on A549 were analyzed with an LSRII flow cytometer (BD Biosciences) with excitation at 488 nm and emission at 525 nm. As can be seen in FIG. 2, the cells treated with alloferon expressed significantly less

The stromal cell-derived factor 1 (SDF1), also known as C-X-C motif chemokine 12 (CXCL12), is a chemokine protein. It is ubiquitously expressed in many tissues and cell types. CXCL12 signaling has been observed in several cancers and is closely related with the metastasis of cancer after interaction with CXCR4 on cancer cells. One hundred thousand of either A549 (FIG. **2(b)**) or H1299 (FIG. **2(c)**) cells were plated in a T25 culture flask and cultured in the presence or absence of alloferon (0.5 mg/ml). Then 100 ng/ml of SDF-1 was added to the cell cultures and incubated for another 12 hours. At the end of that time, culture supernatants were collected and the amount of SDF-1 were measured by ELISA (R&D systems), according to the manufacturer's protocol. Optical density was measured at 405 nm. The data were expressed as mean$\pm$SD for each group of independent experiments. For the comparison of three or more groups, the data were analyzed using the Student's t-test or one-way ANOVA followed by the Newman-Keuls multiple comparison test. A p-value<0.05 was considered statistically significant. The statistical analyses were carried out using the GraphPad InStat (GraphPad Software, San Diego, CA, USA). (* 0.05, ** 0.005. *** 0.001). As can be seen in FIG. 2(b) and 2(c), there is a reduction in SDF-1 secretion that depends on the time period that the cells are cultured in the presence of alloferon.

<EXAMPLE 2>

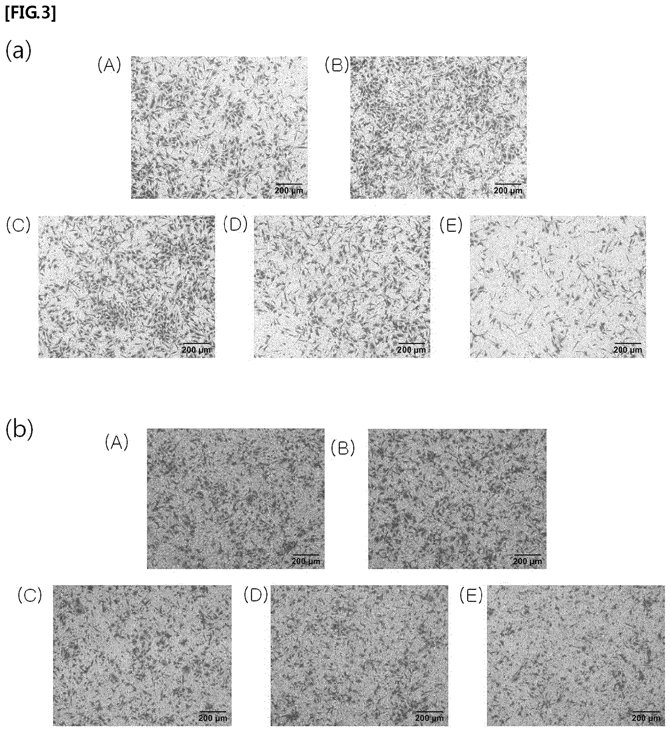

A549 (FIG. **3(a)**) and H1299 (FIG. **3(b)**) cells were used in a migration assay to assess whether alloferon treatment had any effect on the ability of the cells to migrate into a three-dimensional gel as a model for metastasis. Ten thousand A549 cells were pre-treated 0.5 $\mu$g/ml of alloferon for 24, 48 and 96 hours, and then the cells were placed in the upper chamber of 8 micron Transwells in a 24-well plate (Costar Inc.) on 100 $\mu$l of solid growth factor reduced Matrigel (BD Biosciences). Two hundred ng/ml of recombinant SDF was placed in the lower chamber of each well in 24-well plate to induce A549 migration by recombinant SDF or control media in the lower chamber of Transwell inserts with 6.5 $\mu$m pores. After 20 hours in culture at 37°C under 5% $CO_2$, the filter side of the upper chamber was cleaned with a cotton swab and stained for one hour with crystal violet (Sigma) in 2% ethanol and then rinsed in water. The filter was gently cut from the chamber and examined under inverted microscopy to see whether the A549 cells have migrated through the filter pores. For the A549 (FIG. **3(a)**) cell line experiment, the micrographs represent: (A) A549 Control, (B) A549+SDF1, (C) Alloferon (0.5 $\mu$g/ml) pre-treated A549 for 24 hrs+SDF1, (D) Alloferon (0.5 $\mu$g/ml) pre-treated A549 for 48 hrs+SDF1, (E) Alloferon (0.5 $\mu$g/ml) pre-treated A549 for 96 hrs+SDF1. As can be seen in FIG. **3(a),** the longer the pre-treatment in alloferon, the fewer A549 cells migrated into the Matrigel.
For the H1299 (FIG. **3(b)**) cell line experiment, the micrographs represent: (A) H1299 Control, (B) H1299+SDF1, (C) Alloferon (0.5 mg/ml) pre-treated H1299 for 24 hrs+SDF1, (D) Alloferon (0.5 $\mu$g/ml) pre-treated H1299 for 48 hrs+SDF1, (E) Alloferon (0.5 $\mu$g/ml) pre-treated H1299 for 96 hrs+SDF1. Referring to FIG. **3(b),** as with the A549 cells, the longer the pre-treatment in alloferon, the fewer H1299 cells migrated into the Matrigel.

<EXAMPLE 3>

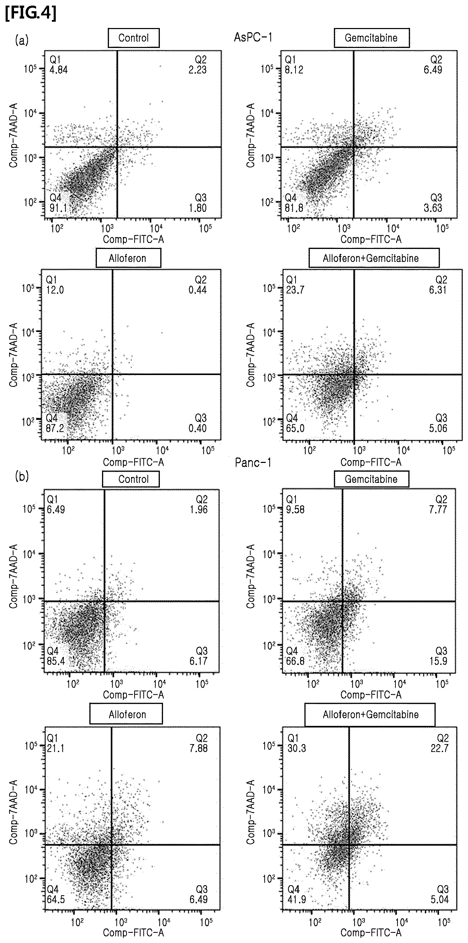

Low concentrations of gemcitabine (250 nM) do not have cytotoxic effects on the ASPC-1 (Fig. 4(a)) and PANC-1 (Fig. 4(b)) pancreatic cancer cell lines and do not induce apoptosis in those cell lines (red circle of each figure). Similarly, 0.5 $\mu$g/ml of alloferon alone does not induce apoptosis in these pancreatic cancer cell lines.
AsPC-1 (FIG **4(a)**) and Panc-1 (FIG **4(b)**), two pancreatic ductal adenocarcinoma cell line, were cultured for week in the presence or absence of 0.5 $\mu$g/ml of alloferon, and then 5 x $10^5$ cells were cultured with 250 nM of gemcitabine for 36 hrs. To investigate whether alloferon increases the sensitivity of these cell lines against gemcitabine, the degree of cell death was examined by an Annexin V-7AAD assay. Cells were collected and washed three times with cold phosphate-buffered saline (PBS) and then resuspended in 1x Annexin V binding buffer at a concentration of 1 x $10^6$ cells/ml (BD Biosciences). Transfer 100 $\mu$l of the solution (1 x $10^5$ cells) to a 5 ml culture tube. Add 5 $\mu$l of FITC-conjugated Annexin V and incubated for 15 min at room temperature in the dark. Add 400 $\mu$l of 1x Annexin V binding buffer to each tube. After 1 $\mu$l of propidium iodide (PI) was added just prior to analyze by flow cytometry. Apoptotic cells were analyzed with LSRII flow cytometer (BD Biosciences) with excitation at 488 nm and emission at 525 nm. As can be seen in FIG. **4,** when gemcitabine is combined with alloferon, the anticancer efficacy can be further enhanced while lowering the toxicity by gemcitabine chemotherapy.

<EXAMPLE 4>

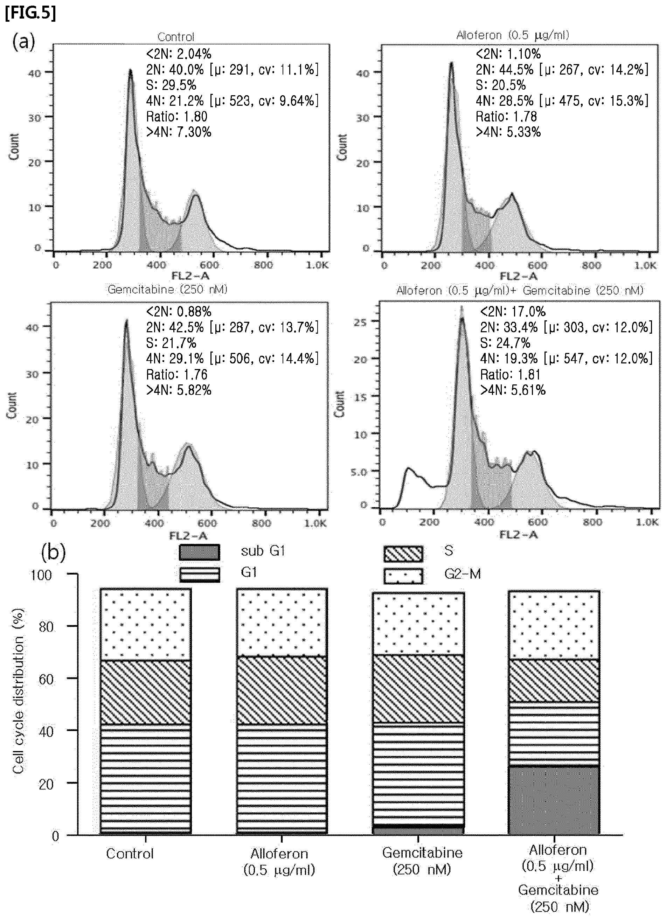

The effect of concomitant alloferon and gemcitabine treatment on the cell cycle distribution of cells was also assessed. AsPC-1 cells were cultured for a week in the presence or absence of 0.5 $\mu$g/ml of alloferon, and then 5 x $10^5$ cells were cultured with 250 nM of gemcitabine for 36 hours. The synergistic effect of alloferon with gemcitabine on the regulation of cell cycle distribution was investigated by 7-AAD (7-Aminoactinomycin D, ThermoFisher Scientific) staining (FIG **5(a)**). The cells were collected and washed three times with PBS and then fixed in cold 70% ethanol for 30 minutes at 4°C. The cells were then washed twice in PBS and treated with 50 $\mu$l of 100 $\mu$g/ml RNase and 20 $\mu$l of 7-AAD. Cells were analyzed with LSRII flow cytometer (BD Biosciences) with excitation at 546 nm and emission at 647 nm. As can be seen in FIG **5(b),** the combined treatment of gemcitabine with alloferon results in a significant increase in cells in apoptosis (sub-G1).

<EXAMPLE 5>

The effect of adjuvant treatment of alloferon and gemcitabine on mitochondrial function was also studied. AsPC-1 cells were cultured for week in the presence or absence of 0.5 mg/ml of alloferon. Then 5 x $10^5$ cells were cultured with 250 nM of gemcitabine for 36 hours. The synergistic effect of alloferon with gemcitabine on the decrease of mitochondrial membrane potential, a general phenomenon associated with cell death by chemotherapeutic drugs, was investigated by staining with DiOC6(3), a green-fluorescent, lipophilic dye (ThermoFisher Scientific). Cells were collected and washed three times with PBS. Then 1 x $10^6$ cells were resuspended in 1 ml freshly prepared 4% paraformaldehyde diluted from stock using PBS for 10 minutes at room temperature and then washed with 1 ml pre-warmed (37°C) PBS three times. The DiOC6 stock solution was diluted into PBS to make 0.1 $\mu$M working solution. The cells were suspended at a density of 1 x $10^6$ cells/ml in dye working solution and protected from light. The cells were incubated at 37°C for 15 minutes. The cell suspensions were centrifuged at 130x g for 5 minutes. The supernatant was removed and the cells were gently resuspended in 1 ml pre-warmed PBS. The cells were analyzed with LSRII flow cytometer (BD Biosciences) with excitation at 488 nm and emission at 525 nm.

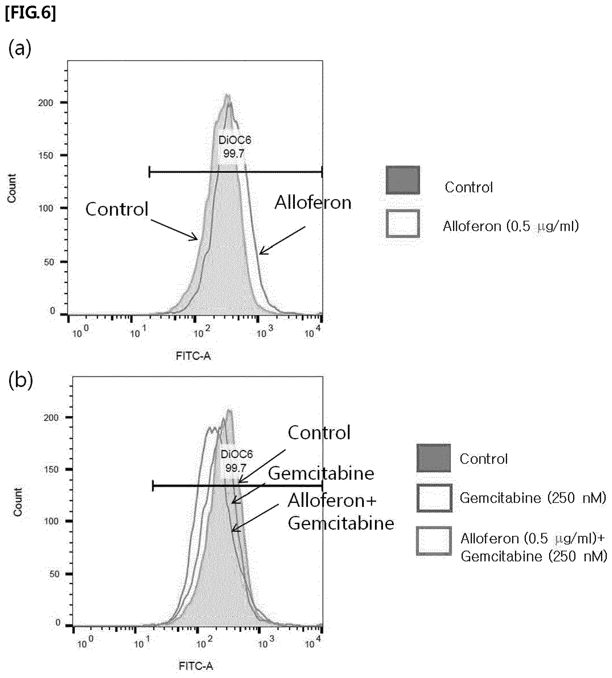

As can be seen in FIG **6(a)** and **6(b),** treatment with alloferon (FIG **6(a))** or gemcitabine (FIG **6(b))** alone does not induce mitochondrial dysfunction as measured by flow cytometry of the mitochondrial membrane potential in pancreatic cancer cells. However, combined treatment of gemcitabine with alloferon induces decrease of mitochondrial membrane potential (FIG **6(b))**, resulting in mitochondrial dysfunction followed by cancer cell death (apoptosis).

<EXAMPLE 6>

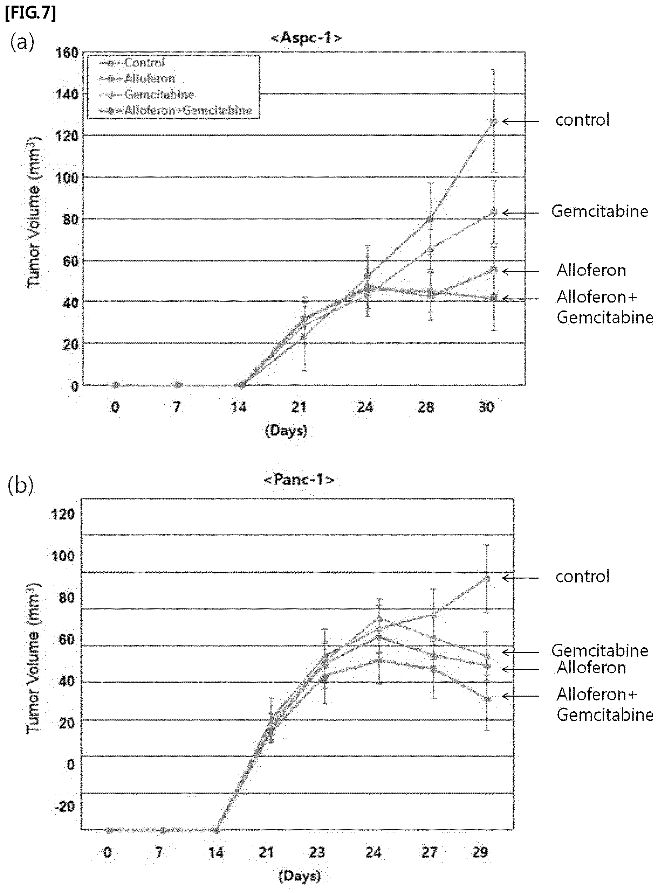

The anti-tumor effect of concomitant alloferon and gemcitabine treatment was also assessed by using xenograft animal model. AsPC-1 cells ($5 \times 10^5$) (FIG 7(a)) and Panc-1 cells ($5 \times 10^5$) (FIG 7(b) were subcutaneoulsly injected at right and left flank of 8 weeks-old female Balb/C (nu/nu) (n=6 per group) obtained from the Jackson Laboratory (Bar Harbor, ME). Tumor size was determined by measuring with calipers every 3-4 days, and the values were inserted into the mathematical equation (1):

[equation 1]

$$\text{Tumor volume (mm}^3) = 0.5 \times \text{(largest diameter)} \times \text{(smallest diameter)}^2$$

As can be seen in FIG **7,** treatment with alloferon or gemcitabine alone does not effectively suppress growth of pancreatic cancer cell lines *in vivo.* However, combined treatment of gemcitabine with alloferon effectively suppresses their growth (orange line in each figure).

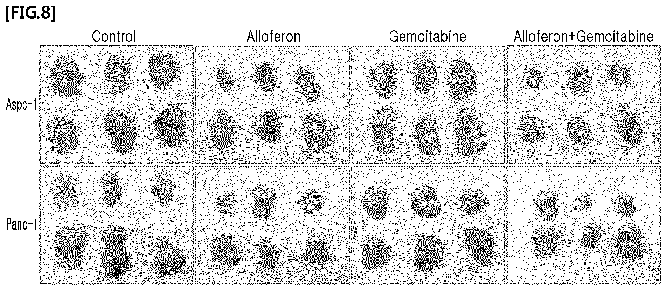

Tumor burdens from each experimental animals were excised and compared its volumes (FIG 8). As shown in tumor growth curve (FIG 7), treatment with alloferon or gemcitabine alone does not effectively suppress growth of pancreatic cancer cell lines, but combined treatment of gemcitabine with alloferon effectively suppresses their growth.

The aforementioned description of the specific embodiments will so fully reveal the general nature of the disclosure that others can, by applying knowledge within the skill of the art, readily modify and/or adapt for various applications such specific embodiments, without undue experimentation, and without departing from the general concept of the present disclosure. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

References in the specification to "one embodiment," "an embodiment," "an exemplary embodiment," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described.

The exemplary embodiments described herein are provided for illustrative purposes and are not limiting. Other exemplary embodiments are possible, and modifications may be made to the exemplary embodiments. Therefore, the specification is not meant to limit the disclosure. Rather, the scope of the disclosure is defined only in accordance with the following claims and their equivalents.

[Sequence List Free Text]

Be included.

**Claims**

1.  A pharmaceutical composition for reducing metastasis and treating pancreatic cancer comprising a compound of formula (1).

    [formula 1]       $X_1$-His-Gly-$X_2$-His-Gly-Val-$X_3$

    (In formula (1),
    wherein $X_1$ is absent or represents at least one amino acid residue, $X_2$ is a peptide bond or represents at least one amino acid residue, and $X_3$ is absent or represents at least one amino acid residue, or a physiologically acceptable salt thereof.)

2.  A pharmaceutical composition for reducing metastasis and treating pancreatic cancer according to Claim 1, wherein the compound of formula (1) comprises peptides having a sequence selected from the group consisting of SEQ ID NO:1 to SEQ ID NO:20.

3.  A pharmaceutical composition for reducing metastasis and treating pancreatic cancer according to Claim 1, which administered in combination with one or more inhibitor of metabolic processes, topoisomerase inhibitor, cytokine, mitosis inhibitor, compound which interacts with nucleic acids, hormone antagonist, or antibody, a combination of any of said antitumor therapeutic agents.

4.  A pharmaceutical composition for reducing metastasis and treating pancreatic cancer according to Claim 1, which improves the anti-cancer activity of gemcitabine.

5.  An adjuvant for treating pancreatic cancer comprising a compound of formula (1).

    [formula 1]       $X_1$-His-Gly-$X_2$-His-Gly-Val-$X_3$

    (In formula (1),
    wherein $X_1$ is absent or represents at least one amino acid residue, $X_2$ is a peptide bond or represents at least one amino acid residue, and $X_3$ is absent or represents at least one amino acid residue, or a physiologically acceptable salt thereof.)

6.  An adjuvant for treating pancreatic cancer according to Claim 5, which intended to be combined with tumor therapy.

7. An adjuvant for treating pancreatic cancer according to Claim 6, wherein the tumor therapy is one or more treating antitumor therapeutic agents or tumor resection by surgery, a combination thereof.

8. An adjuvant for treating pancreatic cancer according to Claim 7, wherein the antitumor therapeutic agent is one or more inhibitor of metabolic processes, topoisomerase inhibitor, cytokine, mitosis inhibitor, compound which interacts with nucleic acids, hormone antagonist, or antibody, a combination of any of said antitumor therapeutic agents.

9. An adjuvant for treating pancreatic cancer according to Claim 7, wherein the antitumor therapeutic agent is gemcitabine.

10. An adjuvant for treating pancreatic cancer according to Claim 5, wherein the compound of formula (1) comprises peptides having a sequence selected from the group consisting of SEQ ID NO:1 to SEQ ID NO:20.

[FIG.1]

[FIG.2]

(a)

(b)

A549_VEGF ELISA
SDF-1 100ng/ml 12hr treatment

(c)

A549_VEGF ELISA
SDF-1 100ng/ml 12hr treatment

[FIG.3]

(a)

(b)

[FIG.4]

[FIG.5]

[FIG.6]

(a)

(b)

[FIG.7]

(a)

<Aspc-1>

(b)

<Panc-1>

[FIG.8]

<table>
<tr><td colspan="2"><b>INTERNATIONAL SEARCH REPORT</b></td><td>International application No.<br><br><b>PCT/KR2019/007117</b></td></tr>
</table>

**A.    CLASSIFICATION OF SUBJECT MATTER**

*A61K 38/08(2006.01)i, A61K 31/7068(2006.01)i, A61P 35/00(2006.01)i, A61P 35/04(2006.01)i*

According to International Patent Classification (IPC) or to both national classification and IPC

**B.    FIELDS SEARCHED**

Minimum documentation searched (classification system followed by classification symbols)
A61K 38/08; A61K 38/02; A61K 38/10; A61K 38/16; A61P 35/00; C07K 14/435; A61K 31/7068; A61P 35/04

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: Alloferon, pancreatic cancer, anti-cancer, metastasis, gemcitabine

**C.    DOCUMENTS CONSIDERED TO BE RELEVANT**

| Category* | Citation of document, with indication, where appropriate, of the relevant passages | Relevant to claim No. |
|---|---|---|
| A | KR 10-2001-0057591 A (KIM, Soo In et al.) 04 July 2001<br>See abstract; claims 1-12; pages 2-3; table 1. | 1-10 |
| Y |  | 1-10 |
| PY | 신문기사 에이티파머 · 서울대의대, 초파리서 추출한 알로페론, 췌장암 폐섬유증에 효과있다. 한국경제신문. 24 July 2018, non-official translation([online article] The AT-PHARMA CO., LTD. · SEOUL NATIONAL UNIVERSITY COLLEGE OF MEDICINE, Alloferon extracted from a drosophila affects pancreatic cancer pulmonary fibrosis. The Korea Economic Daily.) Retrieved from <https://www.hankyung.com/print/201807239579f><br>See the entire document. | 1-10 |
| A | KR 10-2014-0134957 A (ALLOTECH CO., LTD.) 25 November 2014<br>See abstract; claims 1-15. | 1-10 |
| A | BAE, S. et al. The effect of alloferon on the enhancement of NK cell cytotoxi-city against cancer via the up-regulation of perforin/granzyme B secretion. Immunobiology. 2013, vol. 218, pages 1026-1033<br>See abstract; pages 1026, 1031-1032; figures 1-5. | 1-10 |
| A | CHERNYSH, S. et al. Anti-tumor activity of immunomodulatory peptide alloferon-1 in mouse tumor transplantation model. International Immunopharmacology. 2012, vol. 12, pages 312-314<br>See abstract; pages 313-314. | 1-10 |

☒    Further documents are listed in the continuation of Box C.          ☒    See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br><br>18 OCTOBER 2019 (18.10.2019) | Date of mailing of the international search report<br><br>**18 OCTOBER 2019 (18.10.2019)** |
|---|---|
| Name and mailing address of the ISA/KR<br>Korean Intellectual Property Office<br>Government Complex Daejeon Building 4, 189, Cheongsa-ro, Seo-gu,<br>Daejeon, 35208, Republic of Korea<br>Facsimile No. +82-42-481-8578 | Authorized officer<br><br><br>Telephone No. |

Form PCT/ISA/210 (second sheet) (January 2015)

## INTERNATIONAL SEARCH REPORT

International application No.

**PCT/KR2019/007117**

C (Continuation).     DOCUMENTS CONSIDERED TO BE RELEVANT

| Category* | Citation of document, with indication, where appropriate, of the relevant passages | Relevant to claim No. |
|---|---|---|
| A | US 2015-0202250 A1 (BIOVALENCE SDN. BHD.) 23 July 2015<br>See the entire document. | 1-10 |
| A | PENG, Yun-peng et al. Elevation of MMP-9 and IDO induced by pancreatic cancer cells mediates natural killer cell dysfunction. BMC Cancer. 2014, vol. 14, document no. 738, pages 1-12<br>See the entire document. | 1-10 |

Form PCT/ISA/210 (continuation of second sheet) (January 2015)

INTERNATIONAL SEARCH REPORT
Information on patent family members

International application No.

**PCT/KR2019/007117**

| Patent document cited in search report | Publication date | Patent family member | Publication date |
|---|---|---|---|
| KR 10-2001-0057591 A | 04/07/2001 | AT 362485 T | 15/06/2007 |
| | | DE 60034865 T2 | 06/09/2007 |
| | | EP 1114829 A2 | 11/07/2001 |
| | | EP 1114829 A3 | 18/07/2001 |
| | | EP 1114829 B1 | 16/05/2007 |
| | | ES 2283269 T3 | 01/11/2007 |
| | | JP 2001-348399 A | 18/12/2001 |
| | | JP 3564065 B2 | 08/09/2004 |
| | | KR 10-0394864 B1 | 19/08/2003 |
| | | US 2002-0151679 A1 | 17/10/2002 |
| | | US 2004-0138137 A1 | 15/07/2004 |
| | | US 6692747 B2 | 17/02/2004 |
| | | US 7462360 B2 | 09/12/2008 |
| KR 10-2014-0134957 A | 25/11/2014 | WO 2014-185575 A1 | 20/11/2014 |
| US 2015-0202250 A1 | 23/07/2015 | AU 2013-281396 A1 | 05/02/2015 |
| | | EP 2864362 A1 | 29/04/2015 |
| | | SG 11201408280 A | 29/01/2015 |
| | | WO 2014-003537 A1 | 03/01/2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6692747 B
- US 7462360 B
- WO 2005037824 A
- US 6692747 B2
- US 7462360 B2
- WO 2005037824 A2

**Non-patent literature cited in the description**

- **CHERNYSH.** *Proceedings of the National Academy of Science of the United States,* 2002, vol. 99, 12628
- **BAE.** *Immunobiology,* 2013, vol. 218 (8), 1026
- **ROWINSKY.** *Seminars in Oncology,* 1993, vol. 20 (4), 1
- **XIE.** *Diagnostic Pathology,* 2014, vol. 9, 214
- **CHERNYSH.** Proceedings of the National Academy of Science of the United States. 2002, vol. 99, 12628
- Remington's Pharmaceutical Sciences. Lippincott, Williams & Wilkins, 2000